# EUROPÄISCHE PATENTSCHRIFT

(11) **EP 0 860 696 B1**
(45) Veröffentlichungstag und Bekanntmachung des Hinweises auf die Patenterteilung: **07.05.2003**
(21) Anmeldenummer: 98200395.6
(22) Anmeldetag: 09.02.1998
(51) Int. Cl.: G06T 11/00, G01N 23/04

(54) **Röntgenaufnahmeverfahren mit einer Aufnahmeserie aus unterschiedlichen Perspektiven**
Procedure for X-ray imaging with a series of pictures from various viewpoints
Méthode de prise de vues à rayons X avec une série de prises de vues sous différentes perspectives

(30) Priorität: 14.02.1997 DE 19705599
(43) Veröffentlichungstag der Anmeldung: 26.08.1998
(73) Patentinhaber: Philips Corporate Intellectual Property GmbH, 20099 Hamburg (DE); Koninklijke Philips Electronics N.V., 5621 BA Eindhoven (NL)
(72) Erfinder: Koppe, Reiner Heinrich, Dr., Röntgenstrasse 24, 22335 Hamburg (DE); Klotz, Erhard Paul Artur, Röntgenstrasse 24, 22335 Hamburg (DE); Op de Beek, Joh, Röntgenstrasse 24, 22335 Hamburg (DE)
(74) Vertreter: Volmer, Georg, Dipl.-Ing.

(56) Entgegenhaltungen:
- EP-A- 0 492 896
- EP-A- 0 713 678
- LEMIEUX L ET AL: "VOXEL-BASED LOCALIZATION IN FRAME-BASED AND FRAMELESS STEREOTAXY AND ITS ACCURACY" MEDICAL PHYSICS,US,AMERICAN INSTITUTE OF PHYSICS. NEW YORK, Bd. 21, Nr. 8, 1. August 1994 (1994-08-01), Seiten 1301-1310, XP000466011 ISSN: 0094-2405

## Beschreibung

Die Erfindung betrifft ein Röntgenaufnahme-Verfahren, bei dem eine Serie von zweidimensionalen Röntgenaufnahmen angefertigt und digital gespeichert wird, bei denen ein in einem Untersuchungsbereich befindliches Untersuchungsobjekt aus unterschiedlichen Perspektiven auf einen Röntgenbildaufnehmer projiziert wird, wobei aus den Bildwerten der Röntgenaufnahmen für im Untersuchungsbereich befindliche Voxel Voxel-Bildwerte rekonstruiert werden sowie eine Anordnung zur Durchführung des Verfahrens.

Ein Verfahren und eine Anordnung der genannten Art sind aus der EP-A-492 896 bekannt. Dabei werden Röntgenaufnahmen aus Perspektiven gemacht, die sich um 180° (oder mehr) unterscheiden. Gleichwohl sind die dabei gewonnenen Daten nicht vollständig, weil das Röntgenstrahlenbündel nicht die Ränder des Untersuchungsobjekts erfaßt, so daß einige Röntgenaufnahmen Strukturen des Untersuchungsobjektes enthalten, die in anderen Röntgenaufnahmen fehlen.

Zur Rekonstruktion der Voxel-Bildwerte (als Voxel werden Volumenelemente des Untersuchungsbereichs bezeichnet) dient ein iteratives Verfahren, bei dem zusätzlich zu den Daten der Röntgenaufnahmen a priori-Information über die Außenkontur des Untersuchungsobjekts in die Rekonstruktion einfließt. Dieses Verfahren erfordert einerseits einen hohen Rechenaufwand und eignet sich andererseits nicht für Aufnahmen des Gefäßsystems, weil es voraussetzt, daß das Gefäßsystem in sämtlichen Röntgenaufnahmen mit Kontrastmittel gefüllt ist. Diese Voraussetzung läßt sich in der Praxis kaum erfüllen.

Aufgabe der vorliegenden Erfindung ist es, ein Verfahren anzugeben, das sich für die Rekonstruktion von "verdünnten Strukturen" (dilute structures) eignet, d.h. von Strukturen, die - wie ein mit Kontrastmittel gefülltes Gefäßsystem - nur einen kleinen Teil der Fläche der Röntgenaufnahmen bedecken und die Röntgenstrahlung deutlich stärker absorbieren als der sie umgebende Bereich. Dieses Verfahren soll zu Bildern führen, die - für einen kleinen Teil des Untersuchungsbereichs - die verdünnten Strukturen deutlicher und mit höherem Kontrast darstellen als die ursprünglichen Röntgenaufnahmen.

Diese Aufgabe wird bei einem Verfahren der eingangs genannten Art durch folgende Schritte gelöst:
a) Vorgabe eines Teilvolumens des Untersuchungsbereiches,
b) Ableitung von für die Voxel des Teilvolumens spezifischen Voxel-Bildwerten aus den Bildwerten derjenigen Pixel, auf denen das Voxel in den einzelnen Röntgenaufnahmen abgebildet wird,
c) Projektion der Bildwerte in wenigstens ein synthetisches Projektionsbild

Die Erfindung ermöglich es, verteilte Strukturen, z.B. ein mit Kontrastmittel gefülltes Gefäßsystem, in einem synthetischen Projektionsbild darzustellen, das die in einem vorgebbare Teilvolumen befindlichen Teile des Gefäßsystems deutlicher wiedergibt als die unter unterschiedlichen Perspektiven aufgenommenen Röntgenaufnahmen, aus denen dieses Projektionsbild abgeleitet wird. Dabei werden zunächst die Voxel-Bildwerte bestimmt, die den Voxeln des Teilvolumens zugeordnet sind und aus diesen Voxel-Bildwerten wird ein synthetisches Projektionsbild berechnet, in dem das vorgegebene Teilvolumen abgebildet wird.

Die Erfindung basiert auf folgenden Überlegungen. Durch die Vorgabe eines Teilvolumens des Untersuchungsbereichs wird zunächst der Bereich festgelegt, in dem die verdünnten Strukturen bzw. das Gefäßsystem mit verbesserter Qualität wiedergegeben werden soll. Je kleiner dieser Bereich ist, umso geringer ist die Rechenzeit und umso deutlicher werden die Unterschiede gegenüber den originalen Röntgenaufnahmen. Danach werden die Voxel-Bildwerte rekonstruiert, die den Voxeln des selektierten Teilvolumens zugeordnet sind. Jedes dieser Voxel beeinflußt in jeder der Röntgenaufnahmen die Bildwerte von einem oder mehreren Pixeln (als Pixel wird ein Bildelement eines Bildes bzw. einer Röntgenaufnahme bezeichnet).

Die Bildwerte dieser Pixel werden zwar auch von Voxeln beeinflußt, die im Untersuchungsbereich außerhalb des Teilvolumens liegen, jedoch beeinflußt ein außerhalb liegendes Voxel nur in einer Röntgenaufnahme den Bildwert desselben Pixels wie ein bestimmtes Voxel innerhalb des Teilvolumens. In den anderen Röntgenaufnahmen beeinflußt das außerhalb liegende Voxel die Bildwerte anderer Pixel als das innerhalb des Teilvolumens befindlichen Voxel. Da der Voxel-Bildwert eines Voxels innerhalb des Teilvolumens aber aus den Bildwerten der ihn in einer größeren Anzahl von Röntgenaufnahmen zugeordneten Pixel abgeleitet wird, bedeutet dies, daß der Einfluß des außerhalb des Teilvolumens befindlichen Voxels auf den Voxel-Bildwert, der einem Voxel innerhalb des Teilvolumens zugeordnet wird, nur einen geringen Einfluß hat. Insofern sind die Verhältnisse ähnlich denen bei einer konventionellen Röntgen-Schichtaufnahme, bei denen außerhalb einer Schicht befindliche Voxel in einem Schichtbild nur "verschmiert" bzw. unscharf wiedergegeben werden.

Danach wird ein synthetisches Projektionsbild berechnet, in das die Voxel des vorgegebenen Teilvolumens - und nur diese - projiziert werden. Das so erzeugte Projektionsbild, das die gleichen - oder andere - geometrischen Parameter haben kann wie eine der Röntgenaufnahmen, gibt daher im wesentlichen nur Strukturen aus dem vorgegebenen Teilvolumen wieder. Es stellt das Gefäßsystem innerhalb des Teilvolumens daher deutlicher dar als die ursprünglichen Röntgenaufnahmen, weil in den Röntgenaufnahmen auch noch eine scharfe Abbildung des Gefäßsystems außerhalb des Teilvolumens enthalten ist, die die Interpretation der interessierenden Gefäßstruktur im Teilvolumen stört.

Bei dem erfindungsgemäßen Verfahren kann es erforderlich sein, die für das Teilvolumen ermittelten Voxel-Bildwerte zunächst zu speichern, bevor daraus ein Projektionsbild (oder mehrere) erstellt wird. Wenn das Teilvolumen relativ groß ist, können darin sehr viele Voxel enthalten sein, so daß ein hoher Speicherbedarf für die Voxel-Bildwerte erforderlich ist.

Die Speicherung sämtlicher Voxel-Bildwerte aus dem vorgegebenen Teilvolumen kann aber entfallen, wenn nach einer bevorzugten Weiterbildung das Verfahren mit der folgenden Reihenfolge der Verarbeitungsschritte durchgeführt wird:
a) Vorgabe eines Voxels aus dem Teilvolumen,
b) Bestimmung der Pixel, die in den Röntgenaufnahmen diesem Voxel zugeordnet sind,
c) Ableitung eines für das Voxel spezifischen Voxel-Bildwertes aus den zu diesen Pixeln gehörenden Bildwerten,
d) Projektion des Voxels in wenigstens ein synthetisches Projektionsbild
e) Wiederholung der Schritte a-d für andere Voxel des Teilvolumens.

Nach dem Schritt d), in dem das Voxel in ein oder mehrere synthetische Projektionsbilder projiziert wird, wird der Voxel-Bildwert dieses Voxels für das bzw. die Projektionsbild(er) nicht mehr benötigt, so daß der Voxel-Bildwert nicht (mehr) gespeichert werden muß. Nur der Voxel-Bildwert des jeweils bearbeiteten Voxels muß gespeichert werden.

Eine bevorzugte Weiterbildung der Erfindung sieht vor, daß der für das Voxel spezifische Voxel-Bildwert aus den Bildwerten der dem Voxel zugeordneten Pixel in den einzelnen Röntgenaufnahmen durch Mittelwertbildung abgeleitet wird. Dadurch ist eine sehr einfache Berechnung des Voxel-Bildwertes aus den Bildwerten der dem Voxel zugeordneten Pixel möglich. Voxel-Bildwerte von Voxeln, in denen sich ein mit Kontrastmittel gefülltes Gefäß befindet, werden dabei nahezu korrekt wiedergegeben, weil die Bildwerte der Pixel in sämtlichen Röntgenaufnahmen maßgeblich von diesem Voxel beeinflußt wurden. Voxel hingegen, die den Bildhintergrund (geringe Absorption) darstellen, können verfälscht rekonstruiert werden, wenn auf die zugehörigen Pixel in den einzelnen Röntgenaufnahmen auch Voxel mit hoher Absorption projiziert werden. Dies stört allerdings nicht, weil eine konturlose Verwischung stattfindet, wenn eine ausreichende Zahl (z.B. 80) von Bildern zur Verfügung steht.

Eine andere Weiterbildung besteht darin, daß der für das Voxel spezifische Voxel-Bildwert aus den Bildwerten der dem Voxel zugeordneten Pixel in den einzelnen Röntgenaufnahmen dadurch abgeleitet wird, daß eine Rangfolge dieser Bildwerte nach ihrer Größe erstellt wird und daß ein Bildwert ausgewählt wird, der einen vorgebbaren Rang innerhalb der Folge einnimmt. Wenn dabei der Rang innerhalb der Rangfolge der Bildwerte so gewählt ist, daß er zwischen dem Median-Wert und dem Bildwert liegt, der der größten Absorption entspricht, ergibt sich eine deutliche Abbildung der verdünnten Strukturen in den Projektionsbildern.

In weiterer Ausgestaltung der Erfindung ist vorgesehen, daß bei der Projektion verschiedener Voxel auf ein gemeinsames Pixel des synthetischen Projektionsbildes dem Pixel von diesen Voxeln derjenige Voxel-Bildwert zugeordnet wird, der der geringsten Absorption der Röntgenstrahlung bei der Röntgenaufnahme entspricht. Diese Ausgestaltung führt dazu, daß der Bildwert eines Pixels in dem synthetischen Projektionsbild durch das Voxel bestimmt wird, das die geringste Absorption aufweist. Das ist in der Regel ein Voxel, das eine verdünnte Struktur wiedergibt, so daß sich eine deutliche Abbildung der verdünnten Strukturen in den synthetischen Projektionsbildern ergibt.

In weiterer Ausgestaltung der Erfindung ist vorgesehen, daß das synthetische Projektionsbild - zumindest bereichsweise - einer nichtlinearen Filterung unterzogen wird, bei der alle Bildwerte jenseits eines Grenzwertes z.B. auf einen Maximaloder Minimalwert gesetzt werden, und bei der die übrigen Bildwerte auf den Bereich zwischen dem Minimal- und dem Maximalwert gespreizt werden. Durch geeignete Wahl des Grenzwertes läßt sich dabei erreichen, daß der Bildhintergrund (d.h. die Bildbereiche, in denen keine verdünnte Struktur abgebildet wird) verschwindet, beispielsweise in dem er in dem Projektionsbild als weiß wiedergegeben wird, während die verdünnten Strukturen (Gefäße) in diesem Bild schwarz erscheinen.

In weiterer Ausgestaltung der Erfindung ist vorgesehen, daß zur Darstellung des Gefäßverlaufs bei einem Patienten vor der Anfertigung der Röntgenaufnahmen eine Kontrastmittelinjektion erfolgt zur Erzeugung einer Serie von Röntgenaufnahmen, die das mit Kontrastmittel gefüllte Gefäßsystem des Patienten darstellen. Erst durch die Kontrastmittelinjektion lassen sich die Gefäße als verdünnte Struktur abbilden. Dabei kann in noch weiterer Ausgestaltung vorgesehen sein, daß die Röntgenaufnahmen durch Subtraktion je eines Paares von den Untersuchungsbereich aus der gleichen Perspektive mit bzw. ohne ein Kontrastmittel darstellenden Röntgenbildern erstellt werden. Die Röntgenaufnahmen entsprechen dabei einer Subtraktionsangiographie, wobei der Bildhintergrund durch die Subtraktion praktisch eliminiert wird.

In weiterer Ausgestaltung der Erfindung ist vorgesehen, daß der Bildaufnehmer bei der Anfertigung der Röntgenaufnahmen auf einem Kreisbogen bewegt wird. Die Bewegung des Bildaufnehmers auf einem Kreisbogen, der den Untersuchungsbereich umschließt, läßt sich besonders einfach realisieren.

Eine Anordnung zur Durchführung des erfindungsgemäßen Verfahrens ist versehen mit einer ersten bildgebenden Einrichtung, die einen Röntgenstrahler und einen Röntgenbildaufnehmer umfaßt, die zur Anfertigung einer Serie von zweidimensionalen Röntgenaufnahmen, bei denen das Untersuchungsobjekt aus unterschiedlichen Perspektiven auf den Röntgenbildaufnehmer projiziert wird, in Bezug auf ein Untersuchungsobjekt verstellbar sind, mit Mitteln zum Speichern: der Röntgenaufnahmen und mit programmierbaren Bildverarbeitungsmitteln, die so programmiert sind, daß folgende Bildverarbeitungsoperationen durchgeführt werden:
a) Vorgabe eines Teilvolumens des Untersuchungsbereiches,
b) Ableitung von für die Voxel des Teilvolumens spezifischen Voxel-Bildwerten aus den zu diesen Pixeln gehörenden Bildwerten
c) Projektion der Bildwerte in wenigstens ein synthetisches Projektionsbild

Dabei ist in weiterer Ausgestaltung der Erfindung vorgesehen, daß sie einen C-Bogen umfaßt, an dem der Röntgenstrahler und der Röntgenbildaufnehmer befestigt sind, und daß der C-Bogen auf einer Kreisbahn in eine Vielzahl von Aufnahme-Positionen bewegbar ist (z.B: motorisch). Eine Anordnung dieser Art ist kostengünstig herzustellen.

Die Erfindung wird nachstehend anhand der Zeichnungen näher erläutert. Es zeigen
Fig. 1 eine Anordnung zur Durchführung des erfindungsgemäßen Verfahrens,
Fig. 2 den Ablauf der einzelnen Verarbeitungsschritte bei dem erfindungsgemäßen Verfahren,
Fig. 3 eine Erläuterung der Bestimmung der Voxel-Bildwerte und
Fig. 4 die Projektion von Voxeln in ein synthetisches Projektionsbild

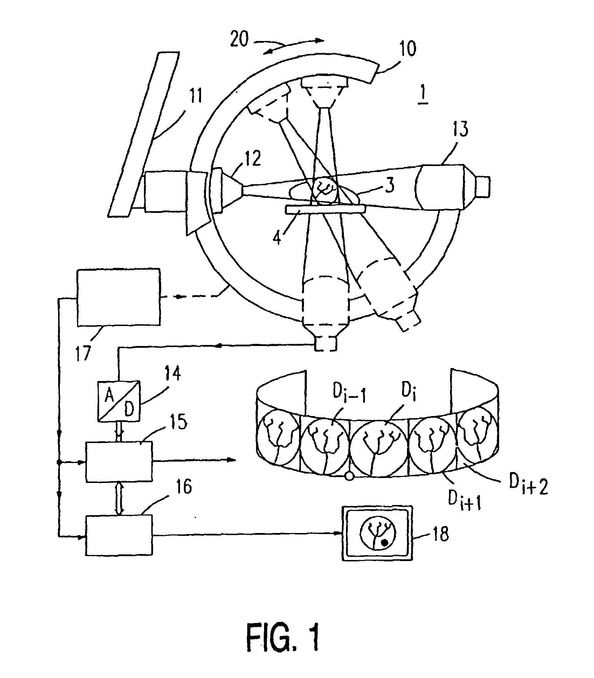

In Fig. 1 ist mit 1 eine bildgebende Einrichtung bezeichnet, die der Erstellung zweidimensionaler Röntgenaufnahmen eines auf einem Tisch 4 befindlichen Untersuchungsobjektes 3 dient, z.B. eines Patienten. Die bildgebende Einrichtung 1 umfaßt einen Röntgenstrahler 12 und einen Röntgenbildaufnehmer 13, die aufeinander ausgerichtetet und an einem Kreisbogen 10 (einem sogenannten C-Bogen) befestigt sind, der seinerseits an einem nur teilweise dargestellten Stativ 11 gehaltert ist. Der C-Bogen 10 kann einerseits um eine waagerechte Achse geschwenkt und andererseits mittels eines nicht näher dargestellten Motorantriebes in Richtung des Doppelpfeiles 20 um z.B. 180° um seinen Mittelpunkt bewegt werden. Bei dieser Bewegung kann eine Vielzahl von Röntgenaufnahmen erzeugt werden, die den Untersuchungsbereich 3, 4 aus verschiedenen - reproduzierbaren - Perspektiven bzw. Winkelpositionen (einige sind gestrichelt angedeutet) des Bildaufnahmesystems 12, 13 abbilden.

Der Röntgenbildaufnehmer 13 kann ein Röntgenbildverstärker mit einer daran angeschlossenen Fernsehkette sein, deren Ausgangssignale von einem Analog-Digital-Wandler 14 digitalisiert und in einem Speicher 15 gespeichert werden, so daß am Ende der Untersuchung die gesamte Röntgenaufnahmeserie gespeichert ist. Diese Röntgenaufnahmen können von einer Bildverarbeitungseinheit 16 verarbeitet werden. Die erzeugten Bilder (.. D₋₁, Dᵢ, Dᵢ₊₁, Dᵢ₊₂ ....) können - einzeln oder als Bildfolge - auf einem Monitor 18 dargestellt werden. Die Steuerung der einzelnen Komponenten der Röntgeneinrichtung erfolgt mit Hilfe einer Steuereinheit 17.

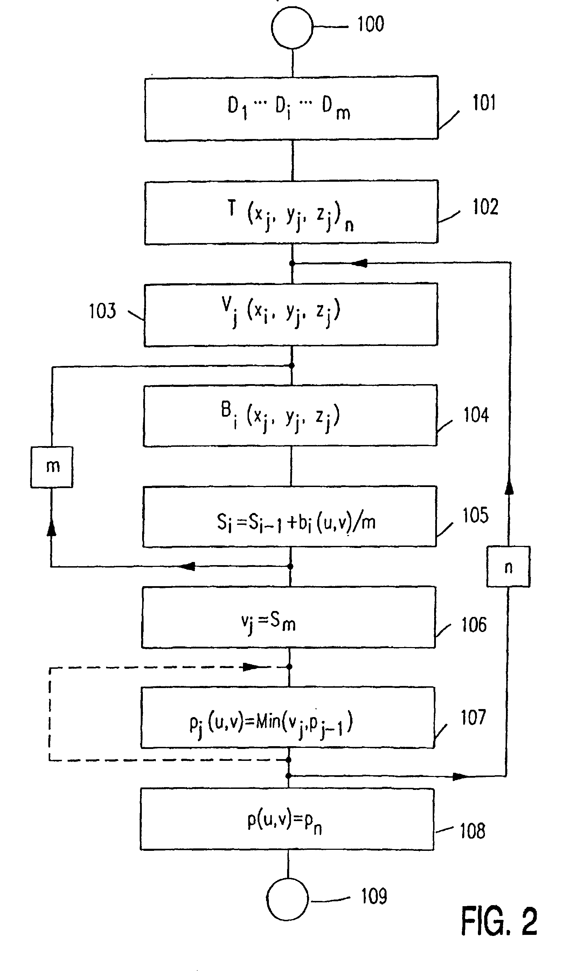

In Fig. 2 ist die Folge der Verfahrensschritte dargestellt, die von der Bildverarbeitungseinheit 16 bzw. der Steuereinheit 17 durchgeführt werden. Nach der Initialisierung (100) wird nach einer Kontrastmittelinjektion der C-Bogen 10 schrittweise um seinen Mittelpunkt bewegt, und gleichzeitig eine Serie von m Röntgenaufnahmen erstellt (z.B. m = 100), die das Untersuchungsobjekt und die darin befindlichen mit einem Kontrastmittel gefüllten Blutgefäße aus unterschiedlichen Perspektiven darstellen (Schritt 101). Dabei ist anzumerken, daß nicht alle Abschnitte des Gefäßsystems bei allen Röntgenaufnahmen mit dem Kontrastmittel gefüllt sind. Das Kontrastmittel kann die betreffenden Abschnitte entweder noch nicht erreicht oder schon wieder verlassen haben. Auch wenn bei der Aufnahmeserie das bildgebende System einen Winkelbereich von 180° oder mehr durchläuft, ergibt sich dann ein Datensatz, der zu einer vollständigen Rekonstruktion (z.B. mit Hilfe einer gefilterten Rückprojektion) nicht ausreicht. Dabei kann noch hinzukommen, daß das bildgebende System zumindest bei einigen Röntgenaufnahmen das Untersuchungsobjekt unvollständig abbildet.

In den Fällen, in denen sich in den Röntgenaufnahmen dem Gefäßsystem lediglich die Abbildung großflächiger Strukturen, z.B. der Schädeldecke, gleichmäßig überlagert, genügt es, eine einzige Röntgenaufnahmeserie anzufertigen. Wenn jedoch kleine Strukturen sich der Abbildung des Gefäßes ungleichmäßig überlagern, z.B. die Halswirbel, empfiehlt es sich, eine Subtraktionsangiographie durchzuführen. Dabei wird vor oder nach der Kontrastmittelinjektion eine weitere Serie von Röntgenaufnahmen erstellt, die dasselbe Objekt unter denselben Perspektiven darstellen - jedoch nicht das Gefäßsystem (weil entweder das Kontrastmittel noch nicht injiziert ist oder das Kontrastmittel sich schon soweit verteilt hat, daß es im Bild nicht mehr sichtbar wird). Anschließend werden die einander entsprechenden Röntgenaufnahmen der beiden Serien voneinander subtrahiert, wobei lediglich das Gefäßsystem verbleibt, weil der Bildhintergrund in beiden Aufnahmeserien der gleiche ist. Die dabei entstehenden Subtraktionsbilder werden im folgenden ebenfalls als Röntgenaufnahmen bezeichnet.

Es kann darüberhinaus zweckmäßig sein, statt einer zwei Röntgenaufnahmeserien anzufertigen, bei denen der C-Bogen jeweils um ± α (z.B. α =30°) bezüglich einer vertikalen Ebene geneigt ist. Damit ist es auch möglich, Strukturen zu erfassen, die bei einer der beiden Röntgenaufnahmeserien in der Ebene des C-Bogens liegen und die sich deshalb nicht darstellen lassen.

In dem ersten Verarbeitungsschritt 101 werden darüberhinaus sämtliche Fehler korrigiert, die durch die Unvollkommenheit des Bildaufnehmers (z.B. Bildschirmkrümmung und andere Bildfehler) oder dadurch bedingt sind, daß der C-Bogen sich durch die Trägheitskraft - reproduzierbar - verformt.

Im folgenden Verarbeitungsschritt 102 wird ein Teilvolumen vorgegeben, das eine für die Diagnose relevante Struktur, z.B. ein Aneurysma enthält. Diese Vorgabe kann interaktiv durch den Benutzer erfolgen, der in zwei vorzugsweise um 90° gegeneinander versetzten Röntgenaufnahmen der Röntgenaufnahmeserie die für die Diagnose relevanten Bereiche z.B. mittels eines Cursors markiert. Das auf diese Weise vorgegebene Teilvolumen T kann n Voxel enthalten, die durch ihre Koordinaten xⱼ, yⱼ, zⱼ im Raum gekennzeichnet sind (1≤j≤n).

Im darauffolgenden Schritt wird zunächst eines der Voxel Vⱼ des Teilvolumens T ausgewählt. Für dieses Voxel wird im Schritt 104 das Pixel Bᵢ bestimmt, das in einer ersten Röntgenaufnahme diesem Voxel zugeordnet ist, d.h. auf das das Voxel bei der Röntgenaufnahme projiziert wird. Eine solche Bestimmung ist möglich, weil einerseits die Lage des Voxels durch die Vorgabe des Teilvolumens definiert ist und weil andererseits die Lage des bildgebenden Systems 12, 13 bei der Anfertigung der Röntgenaufnahmen genau bekannt ist.

In Schritt 105 wird dann aus dem Bildwert bᵢ, das dem ermittelten Pixel zugeordnet ist, dessen Beitrag zum Voxel-Bildwert vⱼ bestimmt, z.B. durch Berechnung des arithmetischen Mittelwertes. Es kann vorkommen, daß die Projektion eines Voxels mehrere Pixel in der Röntgenaufnahme bedeckt; dann sollten deren Bildwerte gemittelt werden. Es kann aber auch vorkommen, daß ein Pixel von der Projektion des Voxels nur teilweise bedeckt wird. Dies kann durch eine entsprechende flächenmäßige Gewichtung des Bildwertes des betreffenden Voxels berücksichtigt werden.

Die aus den Verfahrensschritten 104 und 105 bestehende Schleife wird m mal durchlaufen, wobei die zu dem Voxel gehörenden Pixel jeweils eine andere der m Röntgenaufnahmen bestimmt werden. Nach m-maligem Durchlaufen der Schleife 104, 105 ist dann ein Summenwert bestimmt, der dem arithmetischen Mittelwert der Bildwerte derjenigen Pixel entspricht, die dem Voxel in den verschiedenen Röntgenaufnahmen zugeordnet sind.

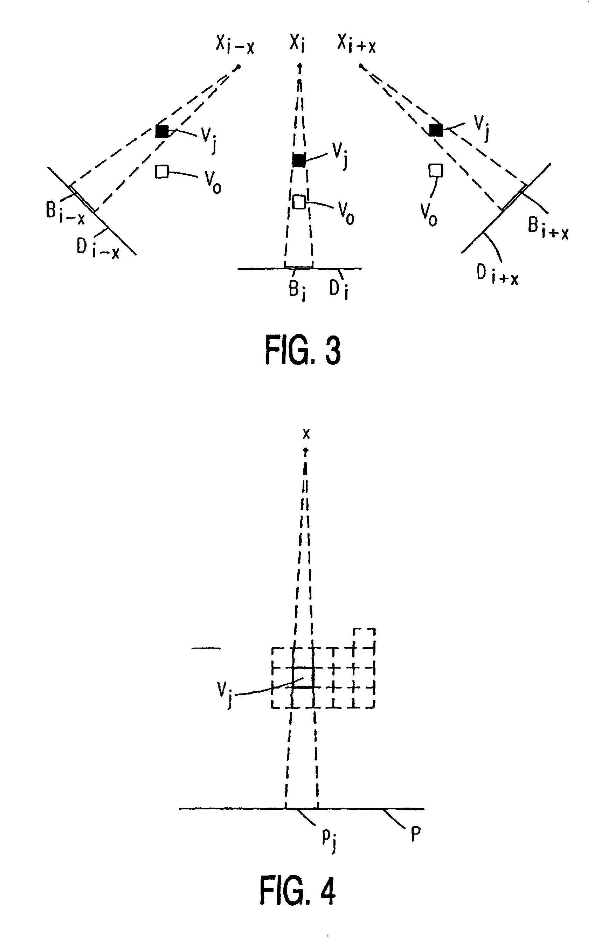

Fig. 3 erläutert diese Verfahrensschritte. Dabei ist für drei Röntgenaufnahmen Dᵢ₋ₓ, Dᵢ und Dᵢ₊ₓ (wobei x ein ganzteiliger Wert sein kann) die Lage des Röntgenstrahlers (Xᵢ₋ₓ, Xᵢ und Xᵢ₊ₓ) dargestellt und - durch die Röntgenaufnahmeebene Dᵢ₋ₓ, Dᵢ und Dᵢ₊ₓ - die zugehörige Lage des Bildaufnehmers. Außerdem ist die Position des selektierten Voxels Vⱼ dargestellt und die Position eines weiteren Voxels Vₒ, das im Abstand von dem Voxel Vⱼ und außerhalb des vorgegebenen Teilvolumens liegt. Weiterhin ist das Pixel (Bᵢ₋ₓ, Bᵢ und Bᵢ₊ₓ) dargestellt, auf das das Voxel in den einzelnen Röntgenaufnahmen projeziert wird.

Man erkennt, daß die Voxel Vⱼ und Vₒ in der Röntgenaufnahme Dᵢ einander überlagert werden. In den anderen Röntgenaufnahmen überlagern sie sich jedoch nicht. Deshalb sind fast alle Bildwerte (bis auf den oder die aus dem Bild Dᵢ abgeleiteten) unabhängig vom Voxel Vₒ. Da der Voxel-Bildwert vⱼ für das Voxel Vⱼ aber aus Bildwerten aus sämtlichen Röntgenaufnahmen abgeleitet wird, bleibt der Einfluß des störenden Voxels Vₒ insgesamt klein.

Der nach m-maligem Durchlaufen der Schleife 104, 105 erhaltene Wert entspricht dem arithmetischen Mittelwert, der im Schritt 106 dem Voxel Vⱼ als Voxel-Bildwert vⱼ zugeordnet wird.

Anstatt den arithmetischen Mittelwert zu berechnen, ist es auch möglich, die zu dem Voxel in den einzelnen Röntgenaufnahmen gehörenden Bildwerte nach ihrer Größe zu ordnen und einen bestimmten Wert in dieser Rangfolge auszuwählen, z.B. den Medianwert oder einen anderen Wert. Allerdings setzt dies voraus, daß sämtliche Bildwerte gespeichert werden müssen, die in den verschiedenen Röntgenaufnahmen dem Voxel Vⱼ zugeordnet sind.

Nachdem auf diese Weise der zu einem Voxel Vⱼ gehörende Voxel-Bildwert vⱼ rekonstruiert wurde, wird ein synthetisches Projektionsbild (oder mehrere Projektionsbilder) berechnet, in das das Voxel Vⱼ (und nachfolgend alle anderen Voxel des Teilvolumens T) projiziert werden. Die Projektionsparameter (d.h. die der Projektion zugrunde gelegte Lage von Röntgenstrahler und Bildaufnehmer) dieses synthetischen Projektionsbildes können mit den Parametern einer der Röntgenaufnahmen identisch sein; jedoch können auch davon abweichende Parameter benutzt werden. Zu diesem Zweck wird in einem Schritt 107 der Beitrag des Voxels Vⱼ, dessen Voxel-Bildwert zuvor ermittelt wurde, zu dem synthetischen Projektionsbild ermittelt. Dazu wird im Schritt 107 zunächst das oder die Pixel ermittelt, auf das das Voxel Vⱼ in dem synthetischen Projektionsbild projiziert wird (vergl. Fig. 4). Für dieses Pixel wird im selben Verfahrensschritt der Beitrag bestimmt, den das Voxel Vⱼ mit seinem Voxel-Bildwert liefert. Dabei können zunächst alle Bildwerte p des Projektionsbildes auf einen Wert gesetzt werden, der einer maximalen Röntgenstrahlungsintensität entspricht ("weiß"). Für das zuvor ermittelte Pixel Pⱼ des synthetischen Projektionsbildes wird dann ein Projektionsbildwert aus dem Vergleich des Voxel-Bildwertes vⱼ mit einem im vorherigen Durchlauf für das betreffende Pixel ermittelten Bildwert pⱼ₋₁ (pₒ entspricht der maximalen Röntgenstrahlenintensität) ermittelt. Entspricht der Voxel-Bildwert vⱼ einer geringeren Röntgenstrahlenintensität, dann wird der Wert pⱼ₋₁ dadurch ersetzt; andernfalls wird pⱼ₋₁ beibehalten.

Wie durch eine gestrichelte Linie angedeutet, wird der Schritt 107 wiederholt, wenn noch ein oder mehrere synthetische Projektionsbilder berechnet werden.

Danach wird die Schleife 103...107 für andere Voxel des Teilvolumens T erneut durchlaufen, und zwar so oft, wie Voxel in dem Teilvolumen vorhanden sind.

Man erkennt, daß einige der Voxel aus dem Teilvolumen das gleiche Pixel Pⱼ projiziert werden, auf das auch das Voxel Vⱼ projiziert wurde. Von den zugehörigen Voxel-Bildwerten vⱼ sämtlicher auf das Pixel Pⱼ projizierten Voxel wird durch das n-fache Ausführen des Schrittes 107 derjenige Voxel-Bildwert dem Pixel im Projektionsbild P zugeordnet, der der minimalen Strahlendosis entspricht. In diesem Fall kann davon ausgegangen werden, daß, wenn überhaupt eines der genannten Voxel einem mit Kontrastmittel gefüllten Gefäß entspricht, letztendlich der Voxel-Bildwert dieses Gefäßes den zugehörigen Projektionsbildwert p bestimmt. Somit ist nach n-maligem Durchlaufen der Verfahrensschritte 103...107 ein synthetisches Projektionsbild P (oder mehrerer solcher Bilder) berechnet, das das Gefäßsystem in dem im Schritt 102 vorgegebenen Teilvolumen T darstellt. Dieses synthetische Projektionsbild P kann auf dem Monitor 18 wiedergegeben werden. Danach ist das Verfahren beendet (Block 109).

Aus den vorstehenden Erläuterungen ergibt sich, daß ein Voxel-Bildwert vⱼ nach dem Verfahrensschritt 107 nicht mehr benötigt wird. Deshalb ist es an sich nicht erforderlich, die Voxel-Bildwerte zu speichern. Stellt sich jedoch heraus, daß die Projektionsparameter für das bzw. die Projektionsbilder so ungünstig gewählt wurden, daß der diagnostisch relevante Bereich nicht klar genug wiedergegeben wird, dann müssen die Voxel-Bildwerte erneut bestimmt werden, wodurch das Rekonstruktionsverfahren verlängert wird. Dies ließe sich dadurch vermeiden, daß sämtliche Voxel-Bildwerte des selektierten Teilvolumens bis zum Ende des Verfahrens gespeichert werden. Allerdings setzt dies einen Speicher voraus, der eine ausreichende Kapazität zur Aufnahme der Voxel-Bildwerte hat. Wenn das im Schritt 102 vorgegebene Teilvolumen T relativ groß ist, ist eine erhebliche Speicherkapazität erforderlich.

Wenn man das Verfahren an einem großen (Partial-)Volumen durchführen will, ergibt sich ein großer Rechenaufwand. Dieser läßt sich dadurch reduzieren, daß dieses in wenigstens zwei Teilvolumina unterteilt wird, und daß das Verfahren für jedes Teilvolumen durchgeführt, wobei die Teilvolumina in ein gemeinsames synthetisches Projektionsbild projeziert werden.

## Patentansprüche

1. Röntgenaufnahme-Verfahren, bei dem eine Serie von zweidimensionalen Röntgenaufnahmen (D₁...Dᵢ...Dₙ) angefertigt und digital gespeichert wird, bei denen ein in einem Untersuchungsbereich befindliches Untersuchungsobjekt (3, 4) aus unterschiedlichen Perspektiven auf einen Röntgenbildaufnehmer projiziert wird, wobei aus den Bildwerten (bᵢ) der Röntgenaufnahmen (Dᵢ) für im Untersuchungsbereich befindliche Voxel (Vⱼ) Voxel-Bildwerte (vⱼ) rekonstruiert werden,
**gekennzeichnet durch** folgende Schritte:
a) Vorgabe eines Teilvolumens (T) des Untersuchungsbereiches,
b) Ableitung von für die Voxel (Vⱼ) des Teilvolumens spezifischen Voxel-Bildwerten (vⱼ) aus den Bildwerten (bᵢ) derjenigen Pixel (Bᵢ), auf die das Voxel in den einzelnen Röntgenaufnahmen (Dᵢ) abgebildet wird,
c) Projektion der Bildwerte (vⱼ) in wenigstens ein synthetisches Projektionsbild (P).

2. Röntgenaufnahme-Verfahren nach Anspruch 1,
**gekennzeichnet durch** folgende Verarbeitungsschritte:
a) Vorgabe eines Voxels (Vⱼ) aus dem Teilvolumen (T),
b) Bestimmung der Pixel (Bᵢ), die in den Röntgenaufnahmen (Dᵢ) diesem Voxel zugeordnet sind,
c) Ableitung eines für das Voxel (Vⱼ) spezifischen Voxel-Bildwertes (vⱼ) aus den zu diesen Pixeln gehörenden Bildwerten (bᵢ),
d) Projektion des Voxels in wenigstens ein synthetisches Projektionsbild
e) Wiederholung der Schritte a-d für andere Voxel des Teilvolumens.

3. Röntgenaufnahme-Verfahren nach Anspruch 1,
**dadurch gekennzeichnet, daß** der für ein Voxel (Vⱼ) des Teilvolumens spezifische Voxel-Bildwert (vⱼ) aus den Bildwerten (bᵢ) der dem Voxel zugeordneten Pixel (Bᵢ) in den einzelnen Röntgenaufnahmen durch Mittelwertbildung abgeleitet wird.

4. Röntgenaufnahme-Verfahren nach Anspruch 1,
**dadurch gekennzeichnet, daß** der für das Voxel (Vⱼ) spezifische Voxel-Bildwert (vⱼ) aus den Bildwerten (bᵢ) der dem Voxel in den einzelnen Röntgenaufnahmen zugeordneten Pixel (Bᵢ) dadurch abgeleitet wird, daß eine. Rangfolge dieser Bildwerte nach ihrer Größe erstellt wird und daß ein Bildwert ausgewählt wird, der einen vorgebbaren Rang innerhalb der Folge einnimmt.

5. Röntgenaufnahme-Verfahren nach Anspruch 1,
**dadurch gekennzeichnet, daß** bei der Projektion verschiedener Voxel (V) auf ein gemeinsames Pixel des synthetischen Projektionsbildes (P) dem Pixel von diesen Voxeln derjenige Voxel-Bildwert zugeordnet wird, der der geringsten Absorption der Röntgenstrahlung bei der Röntgenaufnahme entspricht.

6. Röntgenaufnahme-Verfahren nach Anspruch 1,
**dadurch gekennzeichnet, daß** das synthetische Projektionsbild (P) - zumindest bereichsweise - einer nichtlinearen Filterung unterzogen wird, bei der alle Bildwerte jenseits eines Grenzwertes auf einen Maximal- oder Minimalwert gesetzt werden, und bei der die übrigen Bildwerte auf den Bereich zwischen dem Minimal- und dem Maximalwert gespreizt werden.

7. Röntgenaufnahme-Verfahren nach Anspruch 1,
**dadurch gekennzeichnet, daß** zur Darstellung des Gefäßverlaufs bei einem Patienten vor der Anfertigung der Röntgenaufnahmen eine Kontrastmittelinjektion erfolgt zur Erzeugung einer Serie von Röntgenaufnahmen, die das mit Kontrastmittel gefüllte Gefäßsystem des Patienten darstellen.

8. Röntgenaufnahme-Verfahren nach Anspruch 7,
**dadurch gekennzeichnet, daß** die Röntgenaufnahmen (Dᵢ) durch Subtraktion je eines Paares von den Untersuchungsbereich aus der gleichen Perspektive mit bzw. ohne ein Kontrastmittel darstellenden Röntgenbildern erstellt werden.

9. Röntgenaufnahme-Verfahren nach Anspruch 1,
**dadurch gekennzeichnet, daß** der Bildaufnehmer bei der Anfertigung der Röntgenaufnahmen auf einem Kreisbogen - oder mehreren Kreisbögen mit unterschiedlicher Angulierung (α) - bewegt wird.

10. Röntgenaufnahme-Verfahren nach Anspruch 1,
**dadurch gekennzeichnet, daß** zur Abbildung eines größeren Partialvolumens dieses in wenigstens zwei Teilvolumina unterteilt wird, und daß das Verfahren für jedes Teilvolumen durchgeführt, wobei die Teilvolumina in ein gemeinsames synthetisches Projektionsbild projeziert werden.

11. Anordnung zur Durchführung des Verfahrens nach Anspruch 1, mit einer ersten bildgebenden Einrichtung (1) die einen Röntgenstrahler (12) und einen Röntgenbildaufnehmer (13) umfaßt, die zur Anfertigung einer Serie von zweidimensionalen Röntgenaufnahmen (D₁...Dᵢ...Dₙ), bei denen das Untersuchungsobjekt aus unterschiedlichen Perspektiven auf den Röntgenbildaufnehmer projiziert wird, in Bezug auf ein Untersuchungsobjekt verstellbar sind, mit Mitteln (15) zum Speichern der Röntgenaufnahmen und mit programmierbaren Bildverarbeitungsmitteln (16), die so programmiert sind, daß folgende Bildverarbeitunsgoperationen durchgeführt werden:
a) Vorgabe eines Teilvolumens (T) des Untersuchungsbereiches,
b) Ableitung von für die Voxel (Vⱼ) des Teilvolumens spezifischen Voxel-Bildwerten (vⱼ) aus den Bildwerten (bᵢ) derjenigen Pixel (Bᵢ), auf die das Voxel in den einzelnen Röntgenaufnahmen (Dᵢ) abgebildet wird,
c) Projektion der Bildwerte (vⱼ) in wenigstens ein synthetisches Projektionsbild (P).

12. Anordnung nach Anspruch 11,
**dadurch gekennzeichnet, daß** sie einen C-Bogen (10) umfaßt, an dem der Röntgenstrahler (12) und der Röntgenbildaufnehmer (13) befestigt sind, und daß der C-Bogen auf einer Kreisbahn in eine Vielzahl von Aufnahme-Positionen bewegbar ist.

## Claims

1. An X-ray imaging method in which a series of two-dimensional X-ray images (D₁...Dᵢ...Dₙ) is formed and digitally stored, an object to be examined (3, 4) which is situated in an examination zone being projected onto an X-ray image pick-up device from different perspectives and voxel image values (vⱼ) being reconstructed from the image values (bᵢ) of the X-ray images (Dᵢ) for voxels (Vⱼ) present in the examination zone, **characterized in that** it includes the following steps:
a) selecting a sub-volume (T) of the examination zone,
b) deriving voxel image values (vⱼ) which are specific of the voxels (Vⱼ) of the sub-volume from the image values (bᵢ) of the pixels (Bᵢ) on which the voxel is mapped in the individual X-ray images (Dᵢ),
c) projecting the image values (vⱼ) into at least one synthetic projection image (P).

2. An X-ray imaging method as claimed in claim 1, **characterized in that** it includes the following processing steps:
a) selecting a voxel (Vⱼ) from the sub-volume (T),
b) determining the pixels (Bᵢ) associated with this voxel in the X-ray images (Dᵢ),
c) deriving a voxel image value (vⱼ) which is specific of this voxel (Vⱼ) from the image values (bᵢ) associated with these pixels,
d) projecting the voxel into at least one synthetic projection image,
e) repeating the steps a-d for other voxels of the sub-volume.

3. An X-ray imaging method as claimed in claim 1, **characterized in that** the voxel image value (vⱼ) which is specific of a voxel (Vⱼ) of the sub-volume is derived by averaging from the image values (bᵢ) of the pixels (Bᵢ) associated with the voxel in the individual X-ray images.

4. An X-ray imaging method as claimed in claim 1, **characterized in that** the voxel image value (vⱼ) which is specific of the voxel (Vⱼ) is derived from the image values (bᵢ) of the pixels (Bᵢ) associated with the voxel in the individual X-ray images by ordering these image values according to magnitude, an image value being selected which has a selectable rank within said order.

5. An X-ray imaging method as claimed in claim 1, **characterized in that** upon of projection of different voxels (V) on a common pixel of the synthetic projection image (P), the voxel image value of these voxels which corresponds to the lowest absorption of the X-rays during the X-ray exposure is assigned to the pixel.

6. An X-ray imaging method as claimed in claim 1, **characterized in that** the synthetic projection image (P) is at least partly subjected to a non-linear filtering operation during which all image values beyond a limit value are set to a maximum value or a minimum value whereas the remaining image values are spread out across the range between the minimum value and the maximum value.

7. An X-ray imaging method as claimed in claim 1, **characterized in that** in order to reproduce the vascular system of a patient, a contrast medium is injected, prior to the formation of the X-ray images, so as to form a series of X-ray images reproducing the patient's vascular system filled with contrast medium.

8. An X-ray imaging method as claimed in claim 7, **characterized in that** the X-ray images (Dᵢ) are formed by subtraction of a respective pair of X-ray images which reproduce the examination zone from the same perspective, however, with and without a contrast medium, respectively.

9. An X-ray imaging method as claimed in claim 1, **characterized in that** the image pick-up device is moved along an arc of circle, or along several arcs of circle with a different angulation (á), during the formation of the X-ray images.

10. An X-ray imaging method as claimed in claim 1, **characterized in that** in the case of imaging of a rather large partial volume this volume is subdivided into at least two sub-volumes, and that the method is executed for each sub-volume, the sub-volumes being projected into a common synthetic projection image.

11. A device for carrying out the method claimed in claim 1, including a first imaging device (1) which includes an X-ray source (12) and an X-ray image pick-up device (13) which are adjustable relative to an object to be examined in order to form a series of two-dimensional X-ray images (D₁...Dᵢ...Dₙ) in which the object to be examined is projected onto the X-ray image pick-up device from different perspectives, means (15) for storing the X-ray images and programmable image processing means (16) which are programmed in such a manner that the following image processing operations are carried out:
a) selecting a sub-volume (T) of the examination zone,
b) deriving voxel values (vⱼ) which are specific of the voxels (Vⱼ) of the sub-volume from the image values (bᵢ) of the pixels (Bᵢ) on which the voxel is mapped in the individual X-ray images (Dᵢ),
c) projecting the image values (vⱼ) into at least one synthetic projection image (P).

12. A device as claimed in claim 11, **characterized in that** it is provided with a C-arm (10) whereto the X-ray source (12) and the X-ray image pick-up device (13) are attached, the C-arm being displaceable to a plurality of exposure positions along a circular path.

## Revendications

1. Procédé de radiographie, une série de clichés radiographiques bidimensionnels (D₁..Dᵢ...Dₙ) étant tirée et enregistrée numériquement, un objet d'examen (3, 4) situé dans une zone d'examen étant projeté à partir de différentes perspectives sur un capteur d'images radiographiques, dans lequel des valeurs d'image de voxel (Vⱼ) sont reconstruites à partir des valeurs d'image (Bᵢ) des clichés radiographiques (Dᵢ) pour un voxel (Vⱼ) se trouvant dans la zone d'examen,
**caractérisé par** les étapes suivantes :
a) prédétermination d'un volume partiel (T) de la zone d'examen,
b) déduction de valeurs d'image de voxel (vⱼ) spécifiques pour le voxel (Vⱼ) du volume partiel à partir des valeurs d'image (bᵢ) du pixel (Bᵢ) sur lequel le voxel est représenté dans les différents clichés radiographiques (Dᵢ),
c) projection des valeurs d'image (vⱼ) dans au moins une image de projection synthétique (P).

2. Procédé de radiographie selon la revendication 1,
**caractérisé par** les étapes de traitement suivantes:
a) prédétermination d'un voxel (Vⱼ) à partir du volume partiel (T),
b) détermination des pixels (Bᵢ) qui sont affectés à ce voxel dans les clichés radiographiques (Dᵢ),
c) déduction d'une valeur d'image de voxel (vⱼ) spécifique au voxel (Vⱼ) à partir des valeurs d'image (b;) appartenant à ces pixels,
d) projection du voxel dans au moins une image de projection synthétique,
e) répétition des étapes a-d pour d'autres voxels du volume partiel.

3. Procédé radiographique selon la revendication 1,
**caractérisé en ce que** la valeur d'image de voxel (vⱼ) spécifique pour un voxel (Vⱼ) du volume partiel est dérivée des valeurs d'image (bᵢ) des pixels (Bᵢ) affectés au voxel par calcul d'une moyenne dans les clichés radiographiques individuels.

4. Procédé radiographique selon la revendication 1,
**caractérisé en ce que** la valeur d'image de voxel (vⱼ) spécifique au voxel (Vⱼ) est dérivée des valeurs d'image (bᵢ) des pixels (Bᵢ) affectés au voxel dans les clichés radiographiques individuels par le fait qu'une classification de ces valeurs d'image est établie en fonction de leur taille et qu'une valeur d'image qui adopte un rang à déterminer préalablement dans la séquence est sélectionnée.

5. Procédé radiographique selon la revendication 1,
**caractérisé en ce que**, pour la projection de différents voxels (V) sur un pixel commun de l'image de projection (P) synthétique, il est attribué au pixel de ces voxels la valeur d'image de voxel qui correspond à l'absorption minimale des rayons X lors de la radiographie.

6. Procédé radiographique selon la revendication 1,
**caractérisé en ce que** l'image de projection (P) synthétique est soumise - du moins par zones - à un filtrage non linéaire dans lequel toutes les valeurs d'image au-delà d'une valeur limite sont placées à une valeur maximale ou minimale et les autres valeurs d'image sont dispersées sur la zone entre la valeur minimale et la valeur maximale.

7. Procédé radiographique selon la revendication 1,
**caractérisé en ce que**, pour la représentation du tracé des vaisseaux chez un patient, une injection d'un produit de contraste est effectuée avant la réalisation des clichés radiographiques pour la production d'une série de clichés radiographiques qui représentent le système vasculaire du patient rempli de produit de contraste.

8. Procédé radiographique selon la revendication 7,
**caractérisé en ce que** les clichés radiographiques (Dᵢ) sont réalisés par soustraction respectivement d'une paire d'images radiographiques représentant la zone d'examen à partir de la même perspective avec ou sans produit de contraste.

9. Procédé radiographique selon la revendication 1,
**caractérisé en ce que** le capteur d'images radiographiques est déplacé lors de la réalisation des clichés radiographiques sur un arc de cercle - ou plusieurs arcs de cercle avec un angle (α) différent.

10. Procédé radiographique selon la revendication 1,
**caractérisé en ce que**, pour la représentation d'un volume partiel plus grand, celui-ci est réparti en au moins deux volumes partiels et que le procédé est effectué pour chaque volume partiel, les volumes partiels étant projetés dans une image de projection synthétique commune.

11. Dispositif de mise en oeuvre du procédé selon la revendication 1, avec un premier dispositif d'imagerie (1) qui comprend un générateur de rayons X (12) et un capteur d'images radiographiques (13), lesquels sont déplaçables par rapport à un objet d'examen pour la production d'une série de clichés radiographiques (D₁...Dᵢ....Dₙ) bidimensionnels, l'objet d'examen étant projeté à partir de différentes perspectives sur le capteur d'images radiographiques, avec des moyens (15) pour l'enregistrement des clichés radiographiques et avec des moyens programmables de traitement de l'image (16) qui sont programmés de telle sorte que les opérations de traitement de l'image suivantes soient effectuées :
a) prédétermination d'un volume partiel (T) de la zone d'examen,
b) déduction de valeurs d'image de voxel (vⱼ) spécifiques pour le voxel (Vⱼ) du volume partiel à partir des valeurs d'image (bᵢ) du pixel (Bᵢ) sur lequel le voxel est représenté dans les différents clichés radiographiques (Dᵢ),
c) projection des valeurs d'image (vⱼ) dans au moins une image de projection synthétique (P).

12. Dispositif selon la revendication 11,
**caractérisé en ce qu'**il comprend un arc en C (10) sur lequel sont fixés le générateur de rayons X (12) et le capteur d'images radiographiques (13) et que l'arc en C est mobile sur une trajectoire circulaire dans une multitude de positions d'enregistrement.
